# EUROPEAN PATENT APPLICATION

(11) **EP 4 722 699 A1**
(43) Date of publication of application: **08.04.2026**
(21) Application number: 24815517.8
(22) Date of filing: 29.05.2024
(51) Int. Cl.: G01N 23/20008, C07D 209/20, C07D 401/14, C07F 15/00, C07H 15/14, C07J 19/00, G01N 23/207

(54) **SAMPLE FOR CRYSTAL STRUCTURE ANALYSIS, SAMPLE PRECURSOR FOR CRYSTAL STRUCTURE ANALYSIS, METHOD FOR PRODUCING SAMPLE FOR CRYSTAL STRUCTURE ANALYSIS, AND SAMPLE PREPARATION KIT FOR CRYSTAL STRUCTURE ANALYSIS**

(30) Priority: 30.05.2023 JP 2023088535
(71) Applicant: The University of Tokyo, Bunkyo-ku, Tokyo 113-8654 (JP)
(72) Inventor: FUJITA Makoto, Tokyo 113-8654 (JP); TAKEZAWA Hiroki, Tokyo 113-8654 (JP)
(74) Representative: Wächtershäuser & Hartz Patentanwaltspartnerschaft mbB
(86) International application number: PCT/JP2024/019693
(87) International publication number: WO 2024/248036

(57) **Abstract**

The present disclosure provides a sample precursor for crystal structure analysis including a plurality of host molecules and a plurality of crystallization-promoting molecules, a sample for crystal structure analysis obtained by clathration of target molecules in the sample precursor for crystal structure analysis, a method of producing the sample for crystal structure analysis, and a kit for preparing a sample for crystal structure analysis. The sample precursor for crystal structure analysis is capable of clathration even in cases where the molecule of the compound to be analyzed is a relatively large molecule, a polar molecule, an amphiphilic molecule, or the like.

## Description

### Technical Field

The present disclosure relates to a sample for crystal structure analysis, a sample precursor for crystal structure analysis, a method of producing a sample for crystal structure analysis, and a kit for preparing a sample for crystal structure analysis.

### Background Art

Conventionally, single-crystal structure analysis methods such as single-crystal X-ray diffractometry have been known as highly reliable methods for the determination of a molecular structure. However, in single-crystal structure analysis, a high-quality single crystal needs to be used as a measurement sample. Therefore, in cases where crystal structure analysis is carried out for an unknown compound or a compound that can be hardly crystallized, preparation of the measurement sample has required much time and labor in some cases.

As a method to solve this problem, a method utilizing a single crystal of a porous polymer compound is known.

For example, Patent Literature 1 describes a method in which molecules of a compound to be analyzed are regularly arranged in pores of a single crystal of a polymer complex, and in which crystal structure analysis is carried out using the resulting clathrate of the guest molecules as a measurement sample (the so-called "molecular structure determination method by the crystalline sponge method").

### Citation List

### Patent Literature

Patent Literature 1: International Publication No. WO 2014/038220 (U.S. Patent Application Publication No. 2015/0219533)

### Summary of Invention

### Technical Problem

By using the crystalline sponge method, crystal structure analysis can be carried out even in cases where the amount of the compound to be analyzed is extremely small, or where the compound to be analyzed is a liquid or a gas at room temperature.

However, many of the conventional crystalline sponges (porous polymer compounds) have the problem that clathration is possible only for small molecules, and the problem that they are not suitable for clathration of polar molecules or amphiphilic molecules.

The present disclosure solves these problems, and aims to provide the following: a sample precursor for crystal structure analysis that allows clathration even when the molecule of the compound to be analyzed ("molecule of the compound to be analyzed" may be hereinafter referred to as "target molecule") is a relatively large molecule, a polar molecule, an amphiphilic molecule, or the like; a sample for crystal structure analysis in which the target molecule is clathrated in the sample precursor for crystal structure analysis; a method of producing the sample for crystal structure analysis; and a kit for preparing a sample for crystal structure analysis.

### Solution to Problem

In order to solve the above problem, the present inventors intensively studied molecules having clathration ability. As a result, the present inventors discovered the following:
(i) a single crystal containing three-dimensionally regularly assembled host molecules each including one or more openings, one or more wall portions, and an internal space surrounded by the one or more wall portions includes, as spaces occupiable by target molecules, not only the internal space of each host molecule, but also spaces between the host molecules, so that, by utilizing both spaces, the single crystal can accommodate target molecules such as relatively large molecules, polar molecules, and amphiphilic molecules; and
(ii) by allowing, in a solution, the host molecules to coexist with molecules having a structure capable of affinity interaction with the wall portions of the host molecules, a single crystal in which the host molecules are three-dimensionally regularly assembled can be more readily precipitated;
thereby completing the present disclosure.

Thus, the present disclosure provides the samples for crystal structure analysis according to [1] to [9], the sample precursor for crystal structure analysis according to [10], the methods of producing a sample for crystal structure analysis according to [11] to [13], and the kit for preparing a sample for crystal structure analysis according to [14] as follows.
[1] A sample for crystal structure analysis comprising:
   a plurality of host molecules;
   a plurality of crystallization-promoting molecules; and
   a plurality of target molecules, wherein
   each host molecule includes one or more openings, one or more wall portions, and an internal space surrounded by the one or more wall portions,
   each crystallization-promoting molecule has a structure capable of affinity interaction with a wall portion of the host molecule,
   the plurality of host molecules contained in the sample for crystal structure analysis is three-dimensionally regularly assembled,
   each of the plurality of crystallization-promoting molecules contained in the sample for crystal structure analysis is interposed between wall portions of two host molecules,
   some or all of the plurality of host molecules contained in the sample for crystal structure analysis accommodate, in the respective internal spaces thereof, a whole target molecule or part of a target molecule, and
   the plurality of target molecules contained in the sample for crystal structure analysis is three-dimensionally regularly arranged.
[2] The sample for crystal structure analysis according to [1], wherein each host molecule is a polynuclear metal complex.
[3] The sample for crystal structure analysis according to [2], wherein the polynuclear metal complex contains an ion of an element selected from a group consisting of Ti, Fe, Co, Ni, Cu, Zn, Ru, Rh, Pd, Cd, Os, Ir, and Pt, and a multidentate ligand having a π-conjugated system.
[4] The sample for crystal structure analysis according to claim 3, wherein the multidentate ligand having a π-conjugated system is a ligand represented by Formula (1) below: 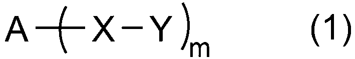 (wherein A is an m-valent group having aromaticity; X is a divalent organic group, or a single bond directly linking A to Y; Y is a coordinating atom, or a monovalent group containing a coordinating atom; m represents an integer of 2 to 6; a plurality of Xs may be different from each other; and a plurality of Ys may be different from each other).
[5] The sample for crystal structure analysis according to any one of [1] to [4], wherein the affinity interaction between the crystallization-promoting molecule and the wall portion of the host molecule is hydrophobic interaction, π-π interaction, or CH-π interaction.
[6] The sample for crystal structure analysis according to any one of [1] to [5], wherein the crystallization-promoting molecule is a molecule having a π-conjugated system.
[7] The sample for crystal structure analysis according to any one of [1] to [6], wherein the crystallization-promoting molecule is a molecule containing a fused aromatic ring.
[8] The sample for crystal structure analysis according to any one of [1] to [7], wherein the crystallization-promoting molecule is a molecule containing an anionic group.
[9] The sample for crystal structure analysis according to any one of [1] to [8], wherein
   an overall charge of each host molecule is a positive charge, and an overall charge of each crystallization-promoting molecule is a negative charge; or
   an overall charge of each host molecule is a negative charge, and an overall charge of each crystallization-promoting molecule is a positive charge.
[10] A sample precursor for crystal structure analysis, comprising a plurality of host molecules and a plurality of crystallization-promoting molecules, wherein
   each host molecule includes one or more openings, one or more wall portions, and an internal space surrounded by the one or more wall portions;
   each crystallization-promoting molecule has a structure capable of affinity interaction with a wall portion of the host molecule;
   the plurality of host molecules contained in the sample precursor for crystal structure analysis is three-dimensionally regularly assembled; and
   each of the plurality of crystallization-promoting molecules contained in the sample precursor for crystal structure analysis is interposed between wall portions of two host molecules.
[11] A method of producing the sample for crystal structure analysis according to any one of [1] to [9], comprising a step of allowing the sample precursor for crystal structure analysis according to [10] to coexist with target molecules in a same system in presence of a solvent or absence of a solvent to accommodate the respective target molecules in the internal spaces of some or all of the host molecules contained in the sample precursor for crystal structure analysis.
[12] A method of producing the sample for crystal structure analysis according to any one of [1] to [9], comprising steps of:
   allowing host molecules each including one or more openings, one or more wall portions, and an internal space surrounded by the one or more wall portions to coexist with target molecules in a same system in presence of a solvent or absence of a solvent to accommodate the target molecules in the internal spaces of the host molecules (Step a-I);
   adding crystallization-promoting molecules to the solution obtained by performing Step a-I in the presence of the solvent, to prepare a solution containing: the host molecules accommodating the target molecules; and the crystallization-promoting molecules (Step a-II); or dissolving the host molecules accommodating the target molecules produced by performing Step a-I in the absence of the solvent, and crystallization-promoting molecules, in a solvent to prepare a solution containing: the host molecules accommodating the target molecules; and the crystallization-promoting molecules (Step a-III); and
   precipitating a single crystal containing: the host molecules accommodating the target molecules; and the crystallization-promoting molecules; from the solution obtained in Step a-II or Step a-III (Step a-IV).
[13] A method of producing the sample for crystal structure analysis according to any one of [1] to [9], comprising steps of:
   dissolving host molecules each including one or more openings, one or more wall portions, and an internal space surrounded by the one or more wall portions; target molecules; and crystallization-promoting molecules; in a solvent to prepare a mixed solution (Step b-I); and
   precipitating a single crystal containing: host molecules accommodating the target molecules; and the crystallization-promoting molecules; from the mixed solution obtained in Step b-I (Step b-II).
[14] A kit for preparing a sample for crystal structure analysis, comprising a combination of: a host molecule including one or more openings, one or more wall portions, and an internal space surrounded by the one or more wall portions; and a crystallization-promoting molecule having a structure capable of affinity interaction with a wall portion of the host molecule.

### Advantageous Effects of Invention

The present disclosure provides a sample for crystal structure analysis, a sample precursor for crystal structure analysis, a method of producing a sample for crystal structure analysis, and a kit for preparing a sample for crystal structure analysis.

### Brief Description of Drawings

FIG. 1 is a diagram illustrating a positional relationship among a host molecule, a crystallization-promoting molecule, and a target molecule in the sample for crystal structure analysis obtained in Example 1;
FIG. 2 is a diagram illustrating the molecular structure of a molecule constituting the sample precursor for crystal structure analysis obtained in Example 2 [FIG. 2(a)], and an enlarged view of a part thereof [FIG. 2(b)];
FIG. 3 is a diagram illustrating the molecular structure of molecules constituting the sample for crystal structure analysis obtained in Example 3;
FIG. 4 is a diagram illustrating the molecular structure of molecules constituting the sample for crystal structure analysis obtained in Example 4;
FIG. 5 is a diagram illustrating the positional relationship among a host molecule, a crystallization-promoting molecule, and a target molecule in the sample for crystal structure analysis obtained in Example 5;
FIG. 6 is a diagram illustrating the molecular structure obtained by crystal structure analysis in Example 6;
FIG. 7 is a diagram illustrating the molecular structure obtained by crystal structure analysis in Example 7;
FIG. 8 is a diagram illustrating the molecular structure obtained by crystal structure analysis in Example 8;
FIG. 9 is a diagram illustrating the molecular structure obtained by crystal structure analysis in Example 9;
FIG. 10 is a diagram illustrating the molecular structure obtained by crystal structure analysis in Example 10; and
FIG. 11 is a diagram illustrating the molecular structure obtained by crystal structure analysis in Example 11.

### Description of Embodiments

The present disclosure is described below in detail regarding each of the following items: 1) a sample for crystal structure analysis; 2) a sample precursor for crystal structure analysis; 3) a method of producing a sample for crystal structure analysis; and 4) a kit for preparing a sample for crystal structure analysis.

### 1) Sample for Crystal Structure Analysis

The sample for crystal structure analysis of the present disclosure is a sample for crystal structure analysis comprising: a plurality of host molecules; a plurality of crystallization-promoting molecules; and a plurality of target molecules;
wherein
each host molecule includes one or more openings, one or more wall portions, and an internal space surrounded by the one or more wall portions;
each crystallization-promoting molecule has a structure capable of affinity interaction with a wall portion of the host molecule;
the plurality of host molecules contained in the sample for crystal structure analysis is three-dimensionally regularly assembled;
each of the plurality of crystallization-promoting molecules contained in the sample for crystal structure analysis is interposed between wall portions of two host molecules;
some or all of the plurality of host molecules contained in the sample for crystal structure analysis accommodate, in the respective internal spaces thereof, a whole target molecule or part of a target molecule; and
the plurality of target molecules contained in the sample for crystal structure analysis is three-dimensionally regularly arranged.

In the present description, the term "molecule" includes not only an electrically neutral substance consisting of two or more atoms, but also a charged substance (an ion) consisting of two or more atoms.

The term "host molecule" means a molecule that includes an opening, a wall portion, and an internal space, and that has the ability to accommodate a target molecule in the internal space.

The term "opening" means a portion that functions as an entrance to the internal space.

The term "wall portion" means a portion that separates the inside of the host molecule from the outside.

The term "internal space" means a space surrounded by one or more wall portions. The shape of the internal space is not limited as long as a target molecule can be accommodated therein. For example, the internal space may have an elongated shape similarly to a "pore".

The term "accommodate a target molecule in the internal space" includes not only a state where the whole target molecule is completely accommodated in the internal space, but also a state where part of the target molecule is accommodated in the internal space and the remaining part of the target molecule protrudes from an opening.

The term "crystallization-promoting molecule" means a molecule that functions as a co-crystallizing agent when the molecule is added to a solution in which host molecules are dissolved.

The term "target molecule" means the molecule of a compound to be analyzed.

The expression "a plurality of host molecules is three-dimensionally regularly assembled" and the expression "a plurality of target molecules is three-dimensionally regularly arranged" each mean a state where the molecular structures of the host molecules or the molecular structures of the target molecules can be determined by a crystal structure analysis method.

### [Host molecules]

Each host molecule contained in the sample for crystal structure analysis of the present disclosure is a molecule including one or more openings, one or more wall portions, and an internal space surrounded by the wall portions.

Each opening is a portion that functions as an entrance when a target molecule enters the internal space. As long as passage of the target molecule is possible, the shape and size of the opening are not particularly limited.

When the largest circle inscribed in the opening is assumed, the length of the diameter is, for example, 0.1 to 5 nm, preferably 0.3 to 3 nm.

The number of openings included in each host molecule is usually 1 to 10, preferably 1 to 5.

Each wall portion is a portion that functions as a wall that separates the inside of the host molecule from the outside. The wall portion is also a portion capable of affinity interaction with a crystallization-promoting molecule.

The internal space is a space inside the host molecule, and is a portion that accommodates a target molecule. As long as the accommodation of the target molecule is possible, the size of the internal space is not particularly limited.

The inside of the internal space may be partitioned into a plurality of small spaces by one or more molecular chains or the like.

The overall charge of each host molecule contained in the sample for crystal structure analysis of the present disclosure is a positive charge or a negative charge, or the host molecule is not charged.

From the viewpoint of optimizing the combination of the host molecule with the crystallization-promoting molecule to allow efficient precipitation of the single crystal containing the host molecule, the overall charge of the host molecule is preferably a positive charge or a negative charge.

In cases where the polynuclear metal complex described later is used as the host molecule, the host molecule usually has a positive charge as a whole since the polynuclear metal complex contains many metal ions.

The size of the host molecule is not particularly limited. When the smallest rectangular parallelepiped capable of accommodating the host molecule is assumed, the length of the longest side of the rectangular parallelepiped is, for example, 0.3 to 15 nm, preferably 1 to 10 nm, and the length of the shortest side is, for example, 0.3 to 15 nm, preferably 0.5 to 10 nm.

As the host molecule, a polynuclear metal complex is preferred because its molecular design is relatively easy.

A polynuclear metal complex is a metal complex containing two or more metal ions and a ligand.

Examples of the polynuclear metal complex used as the host molecule include a metal complex having a three-dimensional structure that includes two or more metal ions and a ligand having two or more coordination sites, wherein the metal complex has an internal space.

In this polynuclear metal complex, the wall portion of the host molecule is usually constructed by the ligand and the metal ions.

The metal ions are not particularly limited as long as they are capable of constituting the polynuclear metal complex. As the metal ions, ions of elements selected from the group consisting of Ti, Fe, Co, Ni, Cu, Zn, Ru, Rh, Pd, Cd, Os, Ir, and Pt are preferred. Ions of the elements of Group 8, Group 9, or Group 10 of the periodic table are more preferred.

The valence of each metal ion is not particularly limited, and is usually 1 to 4, preferably 1 to 3, more preferably 2.

Examples of the ligand include: a ligand that constitutes the wall portion of the host molecule (which may be hereinafter referred to as "ligand (α)"); and a ligand that plays another role (for example, adjustment of the charge of the polynuclear metal complex, or suppression of polymerization of the polynuclear metal complex by occupation of vacant coordination sites of the metal ions) (which may be hereinafter referred to as "ligand (β)").

As the ligand (α), a multidentate ligand having a π-conjugated system is preferred. In cases where the ligand (α) is a ligand having a π-conjugated system, affinity interaction with the crystallization-promoting molecule is more likely to occur. Further, in cases where the ligand (α) is a multidentate ligand, a host molecule having an internal space suitable for accommodating a target molecule can be more easily obtained. The multidentate ligand having a π-conjugated system is preferably a multidentate ligand that includes a group having aromaticity as a central backbone. Since a multidentate ligand that includes a group having aromaticity as a central backbone is relatively rigid and highly planar, the structure of the host molecule can be easily maintained.

Examples of the ligand (α) include those represented by Formula (1) below.

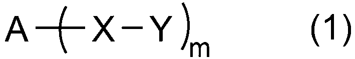

In Formula (1), A represents an m-valent group having aromaticity. X is a divalent organic group, or a single bond directly linking A to Y. Y is a coordinating atom, or a monovalent group containing a coordinating atom. m represents an integer of 2 to 6. A plurality of Xs may be different from each other, and a plurality of Ys may be different from each other.

The number of atoms (excluding hydrogen atoms) in the group represented by A is usually 6 to 100, preferably 6 to 60, more preferably 6 to 30.

Examples of the group represented by A include a six-membered aromatic group; a group in which a plurality of six-membered aromatic groups is linked by single bonds; and a group having a porphyrin skeleton.

Examples of the six-membered aromatic group include a group containing an aromatic ring such as a benzene ring, a triazine ring, a pyridine ring, or a pyrazine ring.

The six-membered aromatic group may contain a substituent other than -(-X-Y).

Examples of the substituent include an alkyl group having 1 to 10 carbon atoms; a halogen atom such as a fluorine atom, a bromine atom, or a chlorine atom; and the like.

Examples of the group represented by A include, but are not limited to, the following. The symbol "*" represents a bonding site (position of bonding to X).

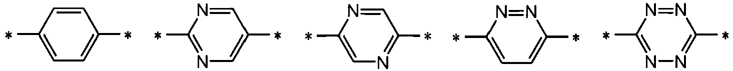

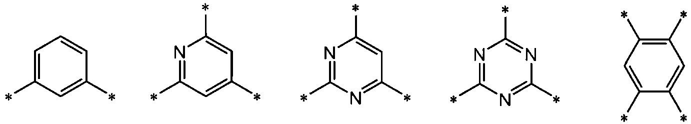

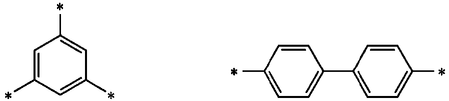

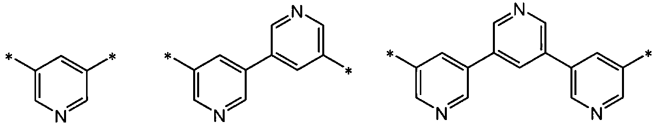

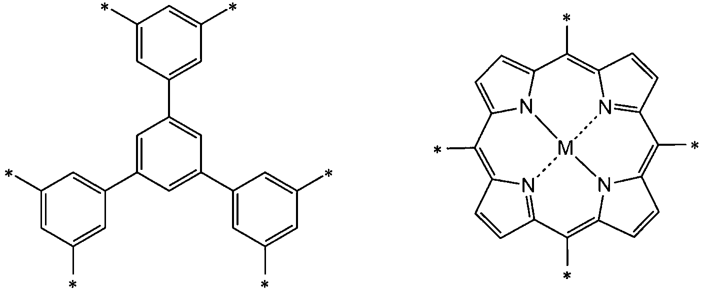

In the above formulae, M represents a metal ion. Examples of the metal ion include those exemplified as the metal ions constituting the polynuclear metal complex. Among these, a zinc ion is preferred.

The number of atoms (excluding hydrogen atoms) in the group represented by X is usually 1 to 30, preferably 2 to 20, more preferably 2 to 10.

Examples of the group represented by X include, but are not limited to: the divalent group represented by A; hydrocarbon groups such as a methylene group, an ethylene group, 1,2-ethenediyl, 1,2-ethynediyl (an acetylene group), a *p*-phenylene group, and an m-phenylene group; an amide group (-C(=O)-NH-); an ester group (-C(=O)-O-); an oxymethylene group (-O-CH₂-); and an oxyethylene group (-O-CH₂CH₂-).

The group represented by X may also be a group in which two or more of these groups are bound together. Examples of such a group include, but are not limited thereto, the following.

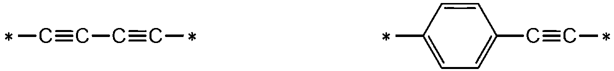

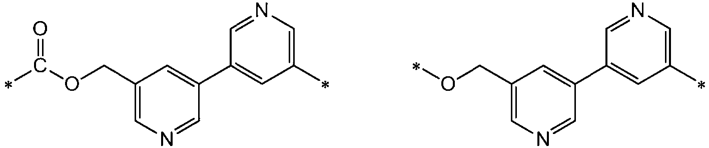

Examples of the coordinating atom represented by Y include an oxygen atom, a sulfur atom, a nitrogen atom, and a phosphorus atom.

The number of atoms (excluding hydrogen atoms) in the monovalent group represented by Y is usually 1 to 20, preferably 1 to 15, more preferably 1 to 10.

Examples of the monovalent group represented by Y include a pyridyl group, an amino group, a hydroxy group, a deprotonated amide group, a carboxylate group, a sulfonate group, a phosphonate group, a dithiocarboxylate group, a cyano group, and groups containing these groups as substituents.

Examples of the monovalent group represented by Y include the following.

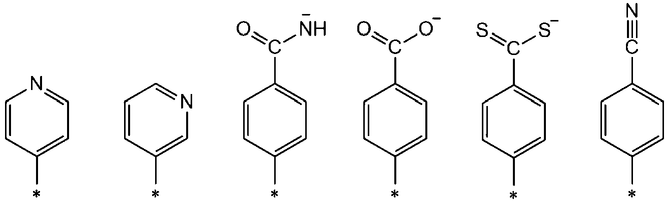

Examples of the ligand (α) include, but are not limited to, the following.

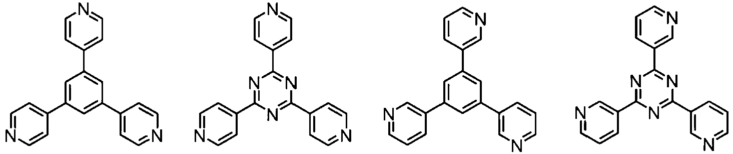

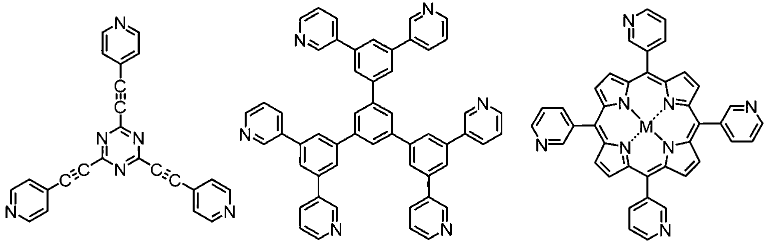

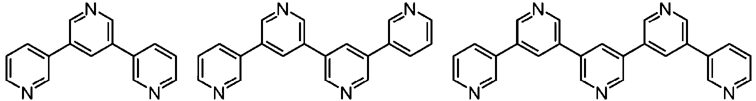

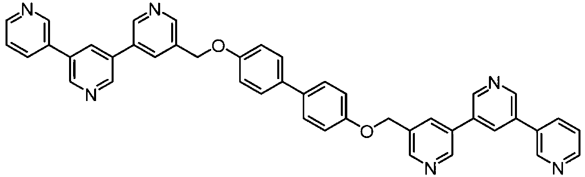

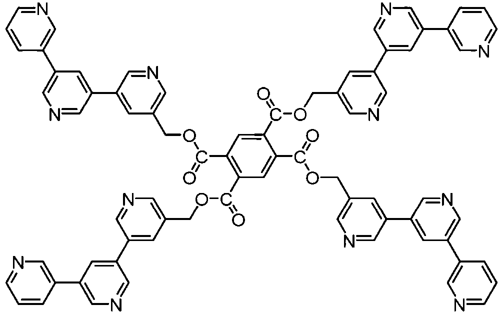

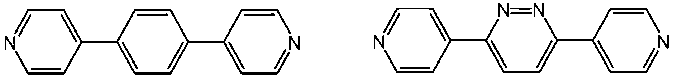

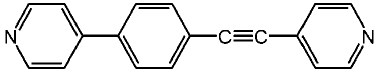

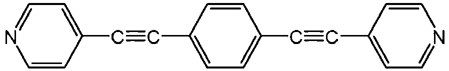

As the ligand (α), ligands other than those represented by Formula (1) can also be used. Examples of such ligands include the following.

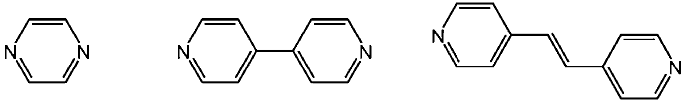

The ligand (β) is preferably a ligand with a relatively low-molecular-weight. In cases where the ligand (β) is a low-molecular-weight ligand, it hardly has an adverse effect on the coordination of the ligand (α) due to steric hindrance or the like.

Examples of the ligand (β) include monodentate ligands and chelating ligands.

Examples of the monodentate ligands used as the ligand (β) include divalent anions such as an oxide ion (O²⁻); monovalent anions such as a hydroxide ion (OH⁻), a chloride ion (Cl⁻), a bromide ion (Br⁻), an iodide ion (I⁻), and a thiocyanate ion (SCN⁻); and electrically neutral coordination compounds such as water, ammonia, monoalkylamines, dialkylamines, and trialkylamines.

Examples of the chelating ligands used as the ligand (β) include, but are not limited to, bidentate chelating ligands such as ethylenediamine, *N,N*'-dimethylethylenediamine, *N,N,N',N'*-tetramethylethylenediamine, 2,2'-bipyridyl, and 1,2-cyclohexanediamine.

By appropriately selecting the metal ion, the ligand (α), and the ligand (β), and using conventionally known methods, a polynuclear metal complex usable as the host molecule can be synthesized.

Known polynuclear metal complexes having clathration ability can also be used as the host molecule.

Examples of such polynuclear metal complexes are shown below together with ligands (α) constituting the polynuclear metal complexes. For expressing the quantitative ratio between the metal ions and the ligands (α) constituting the polynuclear metal complexes, a metal ion is represented as "M"; a ligand (α) is represented as "L"; and, when necessary, a second type of ligand (α) is represented as "X".

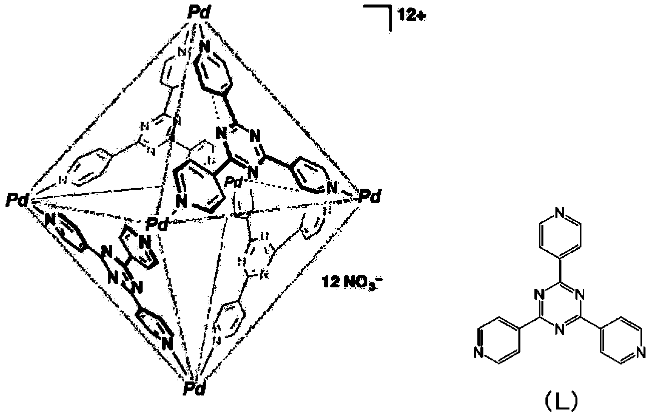

In the above formula, Pd represents a palladium ion to which a bidentate chelating ligand (for example, ethylenediamine) is coordinated. The main component ratio of this polynuclear metal complex is [M₆L₄].

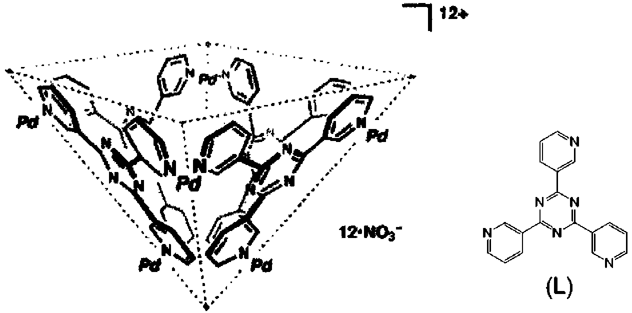

In the above formula, Pd represents a palladium ion to which a bidentate chelating ligand (for example, ethylenediamine) is coordinated. The main component ratio of this polynuclear metal complex is [M₆L₄].

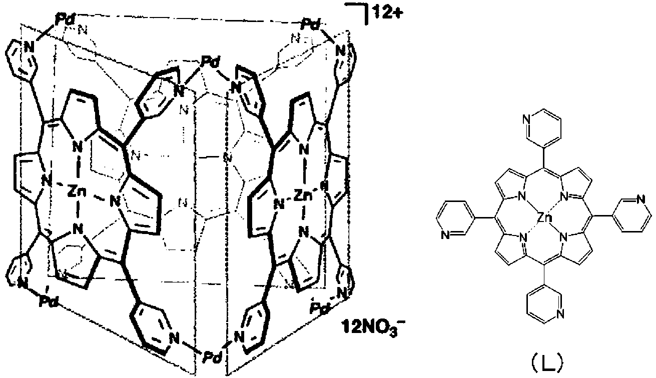

In the above formula, Pd represents a palladium ion to which a bidentate chelating ligand (for example, ethylenediamine) is coordinated. The main component ratio of this polynuclear metal complex is [M₆L₃]. In the ligands represented by L, M represents a metal ion such as a Zn ion.

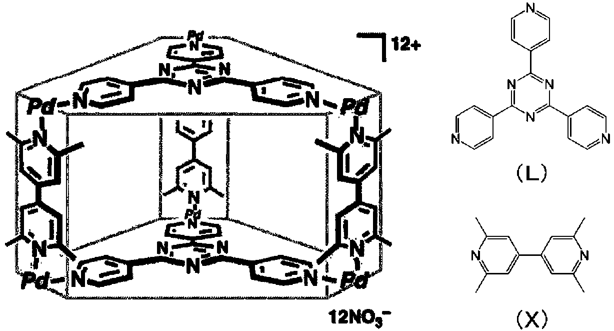

In the above formula, Pd represents a palladium ion to which a bidentate chelating ligand (for example, ethylenediamine) is coordinated. The main component ratio of this polynuclear metal complex is [M₆L₂X₃].

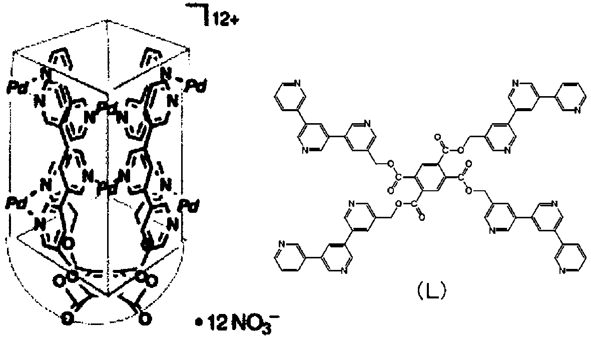

In the above formula, Pd represents a palladium ion to which a bidentate chelating ligand (for example, ethylenediamine) is coordinated. The main component ratio of this polynuclear metal complex is [M₆L₁].

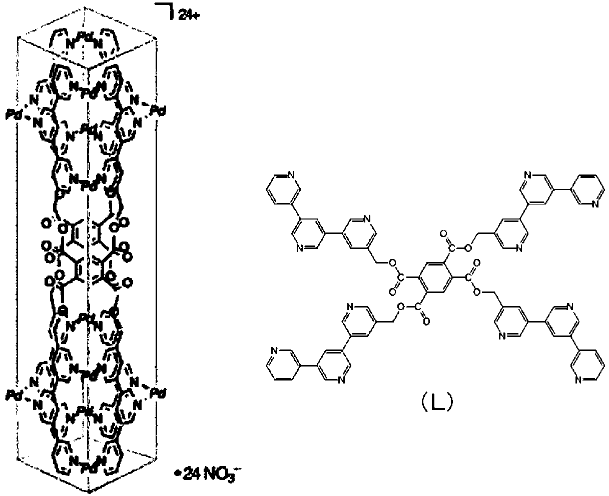

In the above formula, Pd represents a palladium ion to which a bidentate chelating ligand (for example, ethylenediamine) is coordinated. The main component ratio of this polynuclear metal complex is [M₁₂L₂].

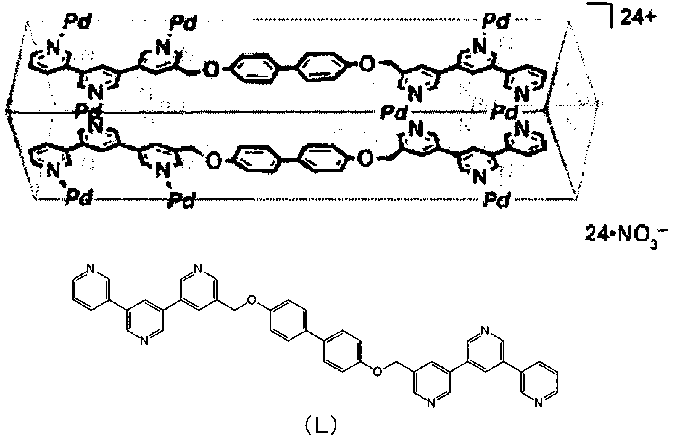

In the above formula, Pd represents a palladium ion to which a bidentate chelating ligand (for example, ethylenediamine) is coordinated. The main component ratio of this polynuclear metal complex is [M₁₂L₄].

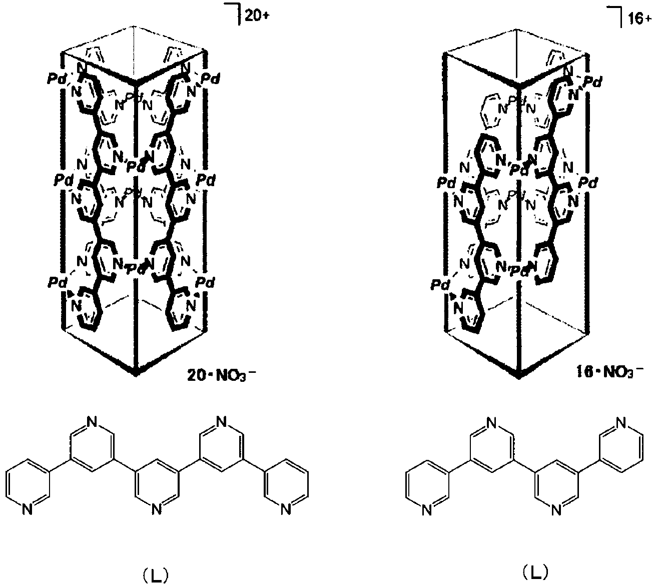

In the above formulae, Pd represents a palladium ion to which a bidentate chelating ligand (for example, ethylenediamine) is coordinated. The main component ratio of the polynuclear metal complex in the left panel is [M₁₀L₄], and the main component ratio of the polynuclear metal complex in the right panel is [M₈L₄].

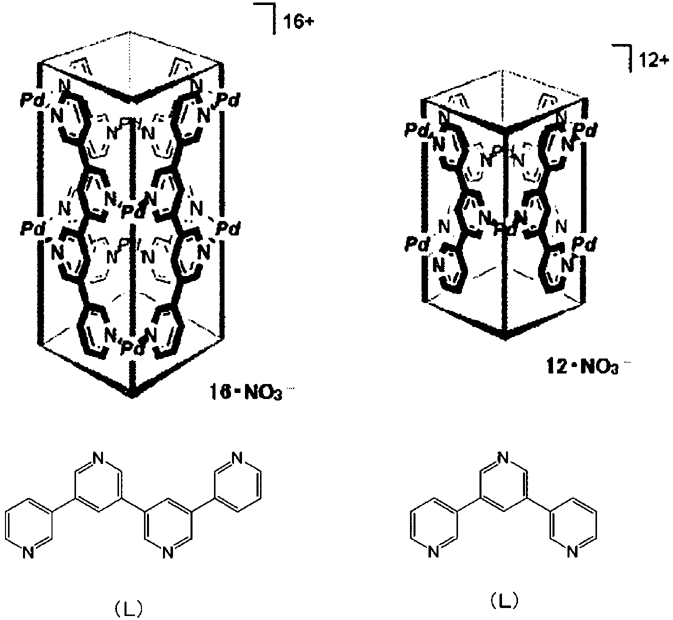

In the above formulae, Pd represents a palladium ion to which a bidentate chelating ligand (for example, ethylenediamine) is coordinated. The main component ratio of the polynuclear metal complex in the left panel is [M₈L₄], and the main component ratio of the polynuclear metal complex in the right panel is [M₆L₄].

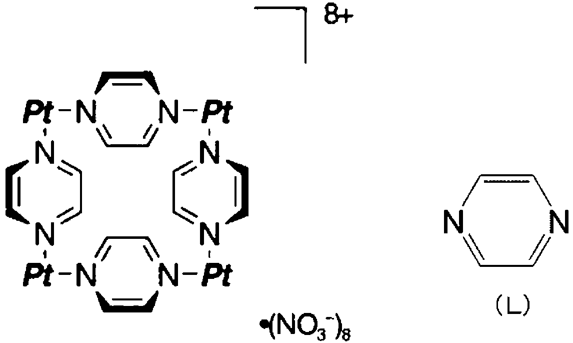

In the above formula, Pt represents a platinum ion to which a bidentate chelating ligand (for example, ethylenediamine) is coordinated. The main component ratio of this polynuclear metal complex is [M₄L₄].

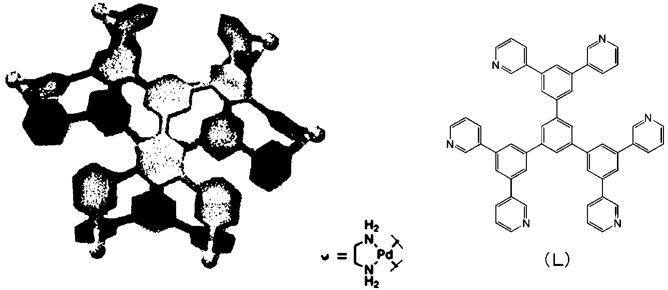

In the above schematic diagram, each portion represented as a sphere represents a palladium ion to which a bidentate chelating ligand (for example, ethylenediamine) is coordinated. The main component ratio of this polynuclear metal complex is [M₆L₂].

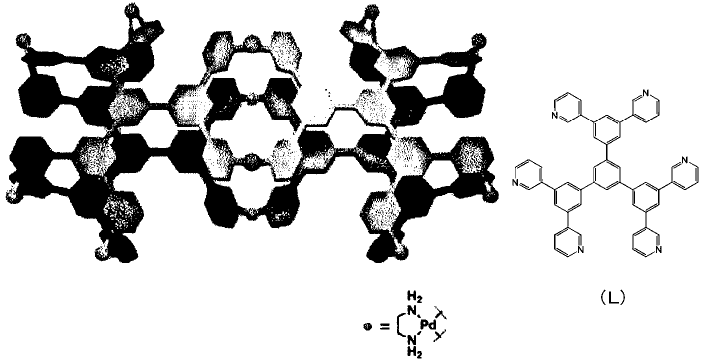

In the above schematic diagram, each portion represented as a sphere represents a palladium ion to which a bidentate chelating ligand (for example, ethylenediamine) is coordinated. The main component ratio of this polynuclear metal complex is [M₁₂L₄].

Details of the methods of synthesizing these polynuclear metal complexes and the like are described in International Publication No. 2018/159692.

### [Crystallization-promoting molecules]

The crystallization-promoting molecules contained in the sample for crystal structure analysis of the present disclosure are molecules having a structure capable of affinity interaction with a wall portion of a host molecule.

Since the crystallization-promoting molecules have such a structure, a single crystal containing host molecules and the crystallization-promoting molecules can be efficiently precipitated by adding the crystallization-promoting molecules to a solution in which the host molecules are dissolved.

Examples of the affinity interaction between the crystallization-promoting molecules and the wall portions of the host molecules include hydrophobic interaction, π-π interaction, and CH-π interaction.

In particular, the affinity interaction between the crystallization-promoting molecules and the wall portions of the host molecules is preferably π-π interaction since this affinity interaction is stronger than the others.

The crystallization-promoting molecules are preferably molecules having a π-conjugated system.

Molecules having a π-conjugated system readily develop π-π interaction and CH-π interaction with the wall portions of the host molecules.

The crystallization-promoting molecules are preferably molecules containing a fused aromatic ring.

Since molecules having a fused aromatic ring tend to exhibit excellent planarity, they are suitable for being interposed between wall portions of two host molecules.

The crystallization-promoting molecules are preferably molecules containing an anionic group. In cases where a host molecule is a polynuclear metal complex, the host molecule tends to have a positive charge as a whole. In such cases, a crystallization-promoting molecule having an anionic group, and having a negative charge as a whole, can function as a counter ion.

Examples of the anionic group include a carboxylate ion group, a sulfonate ion group, and a phosphate ion group. The anionic group is preferably a carboxylate ion group or a sulfonate ion group.

Examples of the crystallization-promoting molecules include molecules having a pyrene skeleton, molecules having a naphthalene skeleton, molecule having a benzene skeleton, and molecules having a biphenyl skeleton.

Examples of the crystallization-promoting molecules having a pyrene skeleton include the molecules represented by the following formulae.

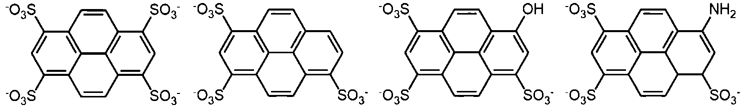

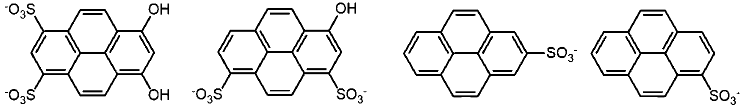

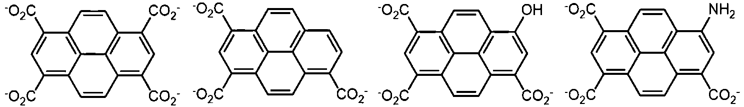

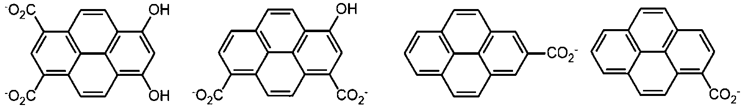

Examples of the crystallization-promoting molecules having a naphthalene skeleton include the molecules represented by the following formulae.

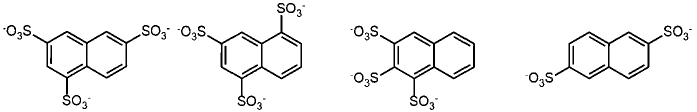

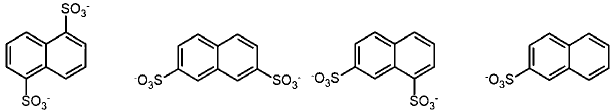

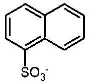

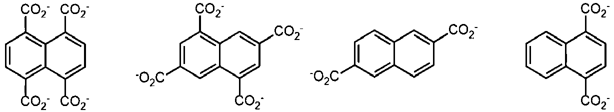

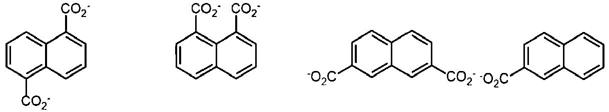

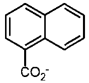

Examples of the crystallization-promoting molecules having a benzene skeleton include the molecules represented by the following formulae.

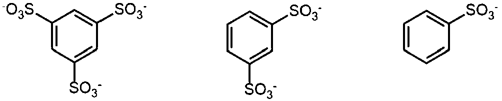

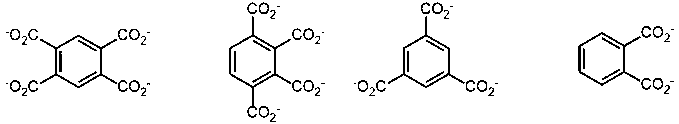

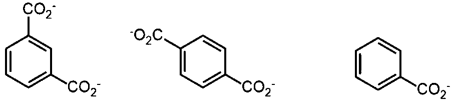

Examples of the crystallization-promoting molecules having a biphenyl skeleton include the molecules represented by the following formulae.

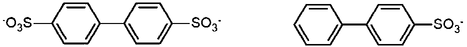

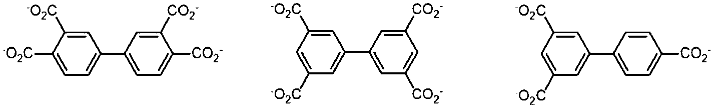

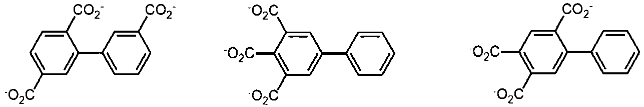

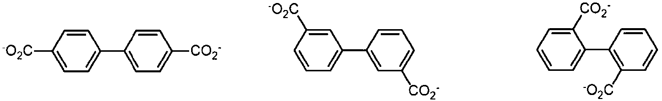

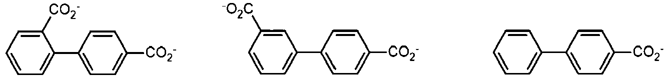

The crystallization-promoting molecules to be used are preferably selected taking into account, for example, the size and shape of the wall portions of the host molecules.

For example, in cases where the multidentate ligands constituting the wall portions are 2,4,6-tris(4-pyridyl)-1,3,5-triazine, those ligands tend to interact with a molecule having an aromatic plane with an equivalent size and a similar shape. Therefore, the crystallization-promoting molecules are preferably molecules having a pyrene skeleton or a naphthalene skeleton. Similarly, in cases where the multidentate ligands constituting the wall portions are 2,4,6-tris(3-pyridyl)-1,3,5-triazine, the crystallization-promoting molecules are preferably molecules having a naphthalene skeleton.

### [Target molecules]

The target molecules contained in the sample for crystal structure analysis of the present disclosure are molecules of a compound whose structural analysis is to be carried out.

The form of the target molecules is not particularly limited, and may be in a solid state, liquid state, or gas state at normal temperature (20°C).

In the sample for crystal structure analysis of the present disclosure, the target molecules can occupy not only the internal spaces of the host molecules, but also the spaces between the host molecules. The internal spaces of the host molecules tend to be hydrophobic, whereas the spaces between the host molecules tend to be hydrophilic.

Accordingly, molecules that have not been suitable as target molecules in the conventional crystalline sponge method (relatively large molecules, polar molecules, amphiphilic molecules, and the like) are also suitable as target molecules constituting the sample for crystal structure analysis of the present disclosure.

The molecular weight of each target molecule is usually not more than 2000, preferably not more than 800. Although there is no particular lower limit of the molecular weight of the target molecule, the molecular weight is usually not less than 50.

### [Sample for crystal structure analysis]

In the sample for crystal structure analysis of the present disclosure, the plurality of host molecules is three-dimensionally regularly assembled. Some or all of these host molecules accommodate, in the respective internal spaces thereof, a whole target molecule or part of a target molecule. The target molecules accommodated in the respective internal spaces of the host molecules are three-dimensionally regularly arranged.

Therefore, by performing crystal structure analysis using the sample for crystal structure analysis of the present disclosure, the molecular structure of the target molecules can be elucidated.

In the sample for crystal structure analysis of the present disclosure, each of the plurality of crystallization-promoting molecules is interposed between wall portions of two host molecules. The crystallization-promoting molecule interposed between the wall portions of the two host molecules contributes to stabilization of the sample for crystal structure analysis.

In the sample for crystal structure analysis of the present disclosure, the host molecules and the crystallization-promoting molecules preferably have charges different from each other.

Such cases include a case where the overall charge of each host molecule is a positive charge and the overall charge of each crystallization-promoting molecule is a negative charge, and a case where the overall charge of each host molecule is a negative charge and the overall charge of each crystallization-promoting molecule is a positive charge.

In cases where the host molecule and the crystallization-promoting molecule have different charges, the crystallization-promoting molecule functions as a counterion of the host molecule, so that the sample for crystal structure analysis can be efficiently produced. Further, a sample for crystal structure analysis in which the host molecule and the crystallization-promoting molecule have different charges has excellent stability.

Further, since a single crystal containing a host molecule and a crystallization-promoting molecule having different charges is often allowed to precipitate from an aqueous solution, the spaces between the host molecules in the single crystal tend to be hydrophilic. Therefore, in the sample for crystal structure analysis in which the host molecule and the crystallization-promoting molecule have different charges, target molecules such as polar molecules and amphiphilic molecules tend to be stably accommodated.

The size of the sample for crystal structure analysis is not particularly limited. The elucidation of the molecular structure of the target molecules is preferably carried out using a sample for crystal structure analysis having a size suitable for the crystal structure analyzer used.

For example, in cases where the crystal structure analysis is carried out using a commercially available X-ray crystal structure analyzer, when the smallest rectangular parallelepiped capable of accommodating the sample for crystal structure analysis is assumed, the length of the long side is usually 10 to 500 µm, preferably 50 to 500 µm, and the length of the short side is usually 10 to 500 µm, preferably 10 to 200 µm.

In cases where a plurality of samples for crystal structure analysis is irradiated with synchrotron radiation X-rays and the molecular structure of the compound to be analyzed is determined based on the collected diffraction intensity data, when the smallest rectangular parallelepiped capable of accommodating the sample for crystal structure analysis is assumed, the length of the long side is usually 0.1 to 100 µm, preferably 1 to 100 µm, and the length of the short side is usually 0.1 to 100 µm, preferably 1 to 10 µm.

In cases where the crystal structure analysis is carried out by the microelectron diffraction method, when the smallest rectangular parallelepiped capable of accommodating the sample for crystal structure analysis is assumed, the length of the long side is usually 0.1 to 1000 nm, preferably 1 to 1000 nm, and the length of the short side is usually 0.1 to 100 nm, preferably 0.1 to 10 nm.

### 2) Sample Precursor for Crystal Structure Analysis

The sample precursor for crystal structure analysis of the present disclosure is a sample precursor for crystal structure analysis comprising a plurality of host molecules and a plurality of crystallization-promoting molecules, wherein each host molecule includes one or more openings, one or more wall portions, and an internal space surrounded by the one or more wall portions; each crystallization-promoting molecule has a structure capable of affinity interaction with a wall portion of the host molecule; the plurality of host molecules contained in the sample precursor for crystal structure analysis is three-dimensionally regularly assembled; and each of the plurality of crystallization-promoting molecules contained in the sample precursor for crystal structure analysis is interposed between wall portions of two host molecules.

The sample precursor for crystal structure analysis of the present disclosure is the same as the sample for crystal structure analysis of the present disclosure except that the target molecules are not accommodated in the internal spaces of the host molecules.

The method of producing the sample precursor for crystal structure analysis of the present disclosure is not particularly limited. For example, by preparing a solution containing host molecules and crystallization-promoting molecules, and leaving the solution to stand to allow precipitation of a single crystal, the sample precursor for crystal structure analysis of the present disclosure can be obtained.

The concentration of the host molecules in the solution containing the host molecules and the crystallization-promoting molecules is usually 0.1 to 50 mM, preferably 5 to 20 mM.

The amount of the crystallization-promoting molecules in the solution containing the host molecules and the crystallization-promoting molecules is usually 0.1 to 10 equivalents, preferably 0.1 to 4 equivalents with respect to the host molecules.

The temperature of the solution to be subjected to the precipitation of the single crystal is usually 0 to 80°C, preferably 4 to 50°C.

The length of time for which the solution is left to stand to allow the precipitation of the single crystal is usually 1 to 240 hours, preferably 1 to 24 hours.

### 3) Method of Producing Sample for Crystal Structure Analysis

The method of producing the sample for crystal structure analysis of the present disclosure is not particularly limited. For example, the sample for crystal structure analysis of the present disclosure can be produced by Production Method (A), Production Method (B), or Production Method (C) below.

### [Production Method (A)]

Production Method (A) comprises a step of allowing the sample precursor for crystal structure analysis to coexist with target molecules in the same system in the presence of a solvent or the absence of a solvent to accommodate the respective target molecules in the internal spaces of some or all of the host molecules contained in the sample precursor for crystal structure analysis.

The term "allowing the sample precursor for crystal structure analysis to coexist with target molecules in the same system" means that these components are placed in a state where they can contact each other. Therefore, the "same system" needs not be a single-phase system as a whole.

Examples of the state where the sample precursor for crystal structure analysis is allowed to coexist with target molecules in the same system in the presence of a solvent include a state where the sample precursor for crystal structure analysis is immersed in a solution or suspension produced by dissolution of at least part of the target molecules in a solvent.

In this case, the immersed sample precursor for crystal structure analysis should not dissolve into the solvent. For example, in the production of the sample for crystal structure analysis of the present disclosure, a solvent in which the sample precursor for crystal structure analysis is insoluble may be used, or the sample precursor for crystal structure analysis may be brought into contact with the target molecules using the mother liquor obtained in the production of the sample precursor for crystal structure analysis.

Examples of the state where the sample precursor for crystal structure analysis is allowed to coexist with the target molecules in the same system in the absence of a solvent include a state where the sample precursor for crystal structure analysis is placed in a container in which gaseous target molecules are present, and a state where the sample precursor for crystal structure analysis is immersed in liquid target molecules.

After the target molecules have contacted the sample precursor for crystal structure analysis, the target molecules are accommodated in the internal spaces of the host molecules constituting the sample precursor for crystal structure analysis. In this process, accommodation of the respective target molecules in a more stable state results in regular arrangement of the plurality of target molecules as a whole.

The amount of the target molecules to be allowed to coexist with the sample precursor for crystal structure analysis is usually not less than 0.1 mol, preferably not less than 0.5 mol per 1 mol of the host molecules.

Conditions for accommodating the target molecules in the internal spaces of the host molecules constituting the sample precursor for crystal structure analysis are not particularly limited.

The temperature at which the target molecules are accommodated in the internal spaces of the host molecules is usually 0 to 100°C, preferably 20 to 100°C.

The length of time for accommodating the target molecules in the internal spaces of the host molecules is usually from 30 seconds to 72 hours, preferably 0.5 to 24 hours.

### [Production Method (B)]

Production Method (B) comprises the steps of:
allowing host molecules each including one or more openings, one or more wall portions, and an internal space surrounded by the one or more wall portions to coexist with target molecules in the same system in the presence of a solvent or absence of a solvent to accommodate the target molecules in the internal spaces of the host molecules (Step a-I);
adding crystallization-promoting molecules to the solution obtained by performing Step a-I in the presence of the solvent, to prepare a solution containing: the host molecules accommodating the target molecules; and the crystallization-promoting molecules (Step a-II); or dissolving the host molecules accommodating the target molecules produced by performing Step a-I in the absence of the solvent, and crystallization-promoting molecules, in a solvent to prepare a solution containing: the host molecules accommodating the target molecules; and the crystallization-promoting molecules (Step a-III); and
precipitating a single crystal containing: the host molecules accommodating the target molecules; and the crystallization-promoting molecules; from the solution obtained in Step a-II or Step a-III (Step a-IV).

Step a-I is a step of accommodating target molecules in the internal spaces of the host molecules by allowing the host molecules to coexist with the target molecules in the same system in the presence of a solvent or in the absence of a solvent.

Step a-I can be carried out in the same manner as in Production Method (A) except that the host molecules are used instead of the sample precursor for crystal structure analysis in Production Method (A).

However, when the host molecules are allowed to coexist with the target molecules in the same system in the presence of a solvent, the host molecules are preferably dissolved in the solvent. In cases where the host molecules are dissolved in the solvent, the host molecules accommodating the target molecules can be produced more efficiently.

In Step a-I, the incorporation of the target molecules in the internal spaces of the host molecules can be confirmed by observing a change in the color or measuring the NMR spectrum.

The amount of the target molecules to be allowed to coexist with the host molecules is usually not less than 0.1 equivalents, preferably not less than 1 equivalent with respect to the host molecules.

Both Step a-II and Step a-III are steps of preparing a solution containing: the host molecules accommodating the target molecules; and the crystallization-promoting molecules.

In cases where Step a-I is carried out in the presence of the solvent, the solution of interest can be prepared by Step a-II.

In cases where the target molecules or the host molecules remain undissolved and are forming a suspension at the end of Step a-I, a filtrate obtained by a filtration process is preferably subjected to the process of Step a-II.

In cases where Step a-I is carried out in the absence of the solvent, the solution of interest can be prepared by Step a-III.

The concentration of the host molecules accommodating the target molecules in the solution prepared in Step a-II or Step a-III is usually 0.1 to 50 mM, preferably 1 to 20 mM.

The amount of the crystallization-promoting molecules in the solution prepared in Step a-II or Step a-III is usually 0.1 to 10 equivalents, preferably 0.1 to 1 equivalents with respect to the host molecules.

Step a-IV is a step of precipitating a single crystal containing: the host molecules accommodating the target molecules; and the crystallization-promoting molecules; from the solution obtained in Step a-II or Step a-III.

The temperature of the solution during the precipitation of the single crystal is usually 0 to 80°C, preferably 4 to 50°C.

The length of time for which the solution is left to stand to allow the precipitation of the single crystal is usually 1 to 240 hours, preferably 1 to 24 hours.

### [Production Method (C)]

Production Method (C) is a method of producing a sample for crystal structure analysis, comprising steps of:
dissolving host molecules each including one or more openings, one or more wall portions, and an internal space surrounded by the one or more wall portions; target molecules; and crystallization-promoting molecules; in a solvent to prepare a mixed solution (Step b-I); and
precipitating a single crystal containing: host molecules accommodating the target molecules; and the crystallization-promoting molecules; from the mixed solution obtained in Step b-I (Step b-II).

Step b-I is a step of preparing a mixed solution by dissolving the host molecules, the target molecules, and the crystallization-promoting molecules in a solvent.

The concentration of the host molecules in the mixed solution is usually 0.1 to 50 mM, preferably 1 to 20 mM.

The amount of the crystallization-promoting molecules in the mixed solution is usually 0.1 to 10 equivalents, preferably 0.1 to 4 equivalents, with respect to the host molecules.

The amount of the target molecules in the mixed solution is usually not less than 0.1 equivalents, preferably not less than 1 equivalent with respect to the host molecules.

Step b-II is a step of precipitating a single crystal containing: the host molecules accommodating the target molecules; and the crystallization-promoting molecules; from the mixed solution obtained in Step b-I.

The temperature of the solution during the precipitation of the single crystal is usually 0 to 80°C, preferably 4 to 50°C.

The length of time for which the solution is left to stand to allow the precipitation of the single crystal is usually 1 to 240 hours, preferably 1 to 24 hours.

Examples of the solvents used in Production Methods (A) to (C) include: aromatic hydrocarbons such as benzene, toluene, xylene, chlorobenzene, 1,2-dichlorobenzene, and nitrobenzene; aliphatic hydrocarbons such as n-pentane, n-hexane, and n-heptane; alicyclic hydrocarbons such as cyclopentane, cyclohexane, and cycloheptane; nitriles such as acetonitrile and benzonitrile; sulfoxides such as dimethyl sulfoxide (DMSO); amides such as N,Ndimethylformamide and n-methylpyrrolidone; ethers such as diethyl ether, tetrahydrofuran, 1,2-dimethoxyethane, and 1,4-dioxane; alcohols such as methanol, ethanol, and isopropyl alcohol; ketones such as acetone, methyl ethyl ketone, and cyclohexanone; Cellosolves such as ethyl Cellosolve; halogenated hydrocarbons such as dichloromethane, chloroform, carbon tetrachloride, and 1,2-dichloroethane; esters such as methyl acetate, ethyl acetate, ethyl lactate, and ethyl propionate; and water. These solvents may be used individually or as a combination of two or more thereof.

### 4) Kit for Preparing Sample for Crystal Structure Analysis

The kit for preparing a sample for crystal structure analysis of the present disclosure comprises the combination of: a host molecule including one or more openings, one or more wall portions, and an internal space surrounded by the one or more wall portions; and a crystallization-promoting molecule having a structure capable of affinity interaction with a wall portion of the host molecule.

The "host molecule" and the "crystallization-promoting molecule" constituting the kit for preparing a sample for crystal structure analysis of the present disclosure are the same as those described in the disclosure of the "sample for crystal structure analysis."

Each of the "host molecule" and the "crystallization-promoting molecule" constituting the kit for preparing a sample for crystal structure analysis of the present disclosure may be in the state of either a pure substance or a solution.

The kit for preparing a sample for crystal structure analysis of the present disclosure may contain two or more kinds of host molecules or two or more kinds of crystallization-promoting molecules, or both.

By using the kit for preparing a sample for crystal structure analysis of the present disclosure, the sample precursor for crystal structure analysis of the present disclosure can be efficiently produced.

By using the kit for preparing a sample for crystal structure analysis of the present disclosure, the sample for crystal structure analysis of the present disclosure can be efficiently produced by Production Method (B) or Production Method (C).

### Examples

The present disclosure is described below in more detail with reference to Examples. However, the present disclosure is not limited to the Examples below.

### [Synthesis Example 1]

### Synthesis of [{(N,N,N',N'-tetramethylethylenediamine)palladium}₆(2,4,6-tris(4-pyridyl)-1,3,5-triazine)₄] Nitrate

According to a method described in J. Am. Chem. Soc. 2004, 126, 9172-9173, nitrate of the octahedral metal complex shown below (which may be hereinafter referred to as "Host Molecule 1") was synthesized.

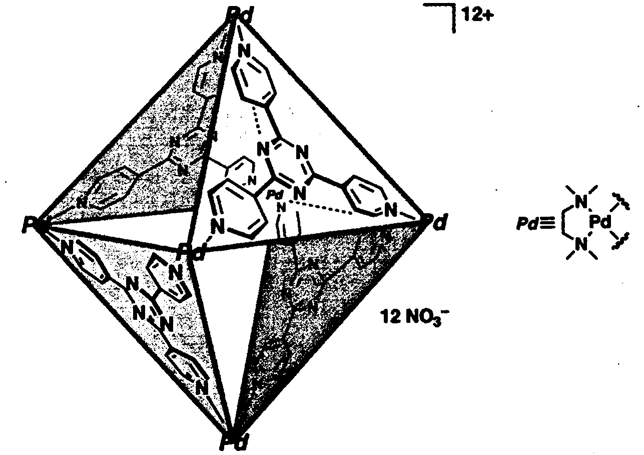

### [Synthesis Example 2]

### Synthesis of [{(N,N,N',N'-tetramethylethylenediamine)platinum}₆(2,4,6-tris(3-pyridyl)-1,3,5-triazine)₄] Nitrate

2,4,6-Tris(3-pyridyl)-1,3,5-triazine (416 mg), (*N,N,N',N'-*tetramethylethylenediamine)platinum nitrato complex (869 mg), and pyrene (215 mg) were suspended in 16 mL of water, and heated with stirring at 100°C for one week. The resulting aqueous solution was washed with dichloromethane, and then freeze-dried to synthesize 969 mg of nitrate of the bowl-shaped metal complex shown below (which may be hereinafter referred to as "Host Molecule 2").

The pyrene used in Synthesis Example 2 serves as a template molecule for synthesizing Host Molecule 2 and is removed from the reaction system by washing with dichloromethane. Therefore, the powder of nitrate of Host Molecule 2 obtained in Synthesis Example 2 does not contain pyrene.

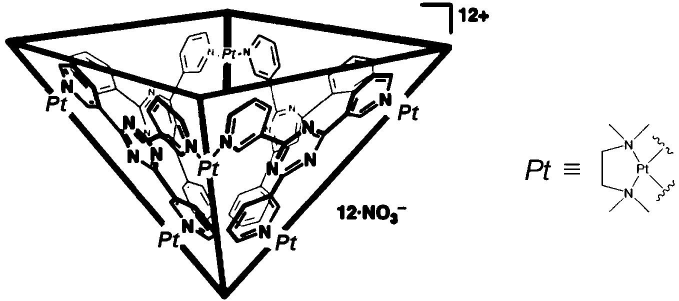

### [Single-Crystal X-Ray Crystal Structure Analysis]

Single-crystal X-ray crystal structure analysis was performed using a Synergy-S diffractometer [Cu-Kα radiation (wavelength, 1.5418 angstroms)] manufactured by Rigaku Corporation.

### [Example 1]

To an aqueous solution of nitrate of Host Molecule 1 (concentration, 10 mM; 200 µL), a powder of digoxin in an amount of 5 equivalents with respect to Host Molecule 1 was added to obtain a suspension. The resulting suspension was stirred at 60°C for 15 minutes, and then filtered using a syringe filter. To the filtrate, an aqueous solution of 1,3,6,8-pyrenetetrasulfonic acid tetrasodium salt (concentration, 20 mM; 10 µL; 0.1 equivalents with respect to Host Molecule 1) was added, and the resulting mixed solution was left to stand at 5°C for 12 hours to allow precipitation of crystals. The precipitated crystals (length of the long side, about 200 µm) were subjected to crystal structure analysis.

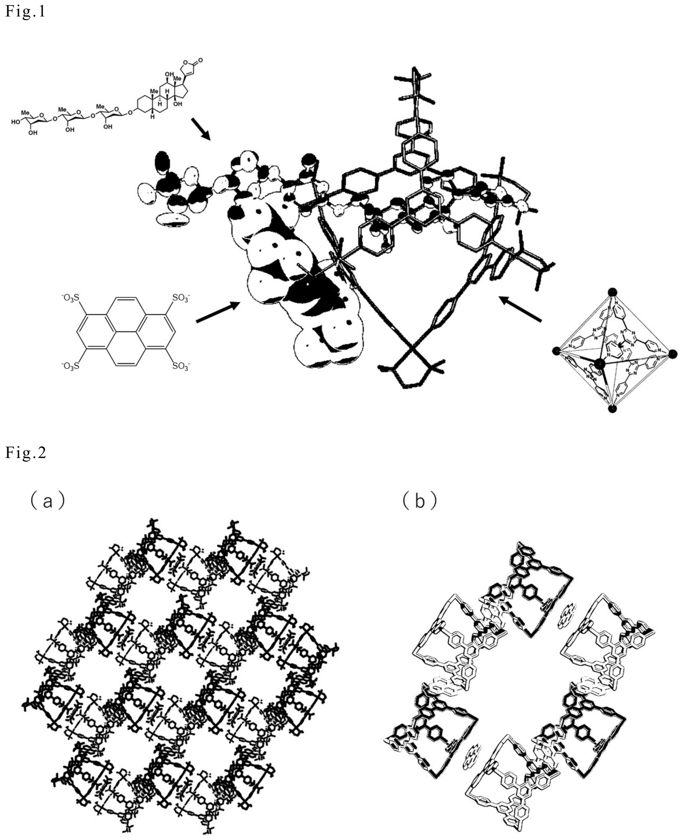

FIG. 1 illustrates the molecular structure obtained by the crystal structure analysis. As illustrated in FIG. 1, the aglycone moiety of the digoxin is accommodated in the internal space of Host Molecule 1, and the sugar moiety of the digoxin protrudes from the internal space of the host molecule to occupy the space formed with the adjacent Host Molecule 1. Further, the framework moiety of 1,3,6,8-pyrenetetrasulfonate ion is oriented almost parallel to a wall portion of Host Molecule 1, and the 1,3,6,8-pyrenetetrasulfonate ion has affinity interaction with the wall portion of Host Molecule 1.

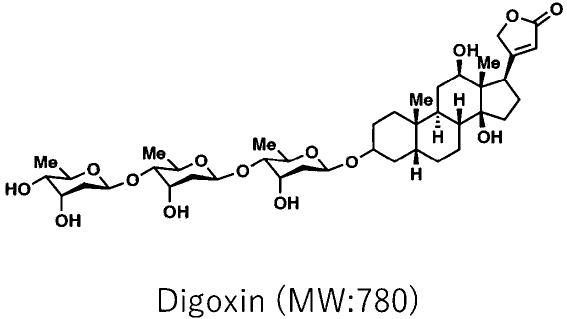

### [Example 2]

To an aqueous solution of nitrate of Host Molecule 1 (concentration, 20 mM; 200 mL), an aqueous solution of 1,3,6,8-pyrenetetrasulfonic acid tetrasodium salt (concentration, 20 mM; 40 µL; 0.2 equivalents with respect to Host Molecule 1) was added, and the resulting mixture was left to stand at 20 °C for 10 hours to allow precipitation of crystals. The precipitated crystals (length of the long side, about 200 µm) were subjected to crystal structure analysis.

FIG. 2(a) illustrates the molecular structure obtained by the crystal structure analysis. An enlarged view of part of the molecular structure is illustrated in FIG. 2(b).

As illustrated in FIG. 2, each 1,3,6,8-pyrenetetrasulfonate ion is interposed between wall portions of two Host Molecules 1. In FIG. 2(b), the sulfonate ion groups of the 1,3,6,8-pyrenetetrasulfonate ion are omitted.

### [Example 3]

Crystals were synthesized in the same manner as in Example 2. From the synthesized crystals, about 50 crystals were taken out together with 10 µL of the mother liquor. To the mother liquor in which the crystals were immersed, 0.1 mg of *n*-octyl-β-D-1-thioglucopyranoside was added, and the resulting mixture was left to stand at 20 °C for 12 hours. Subsequently, the immersed crystals were taken out and subjected to crystal structure analysis.

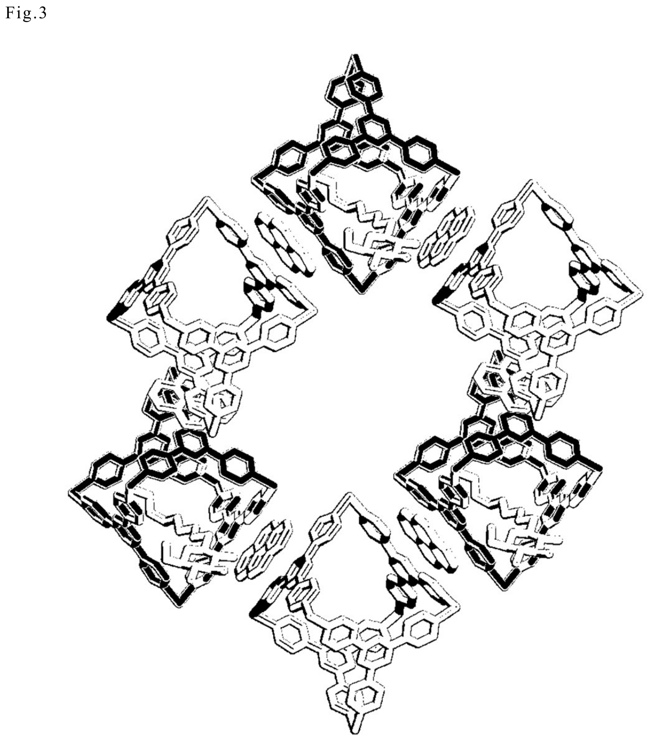

FIG. 3 illustrates the molecular structure obtained by the crystal structure analysis. In FIG. 3, the sulfonate ion groups of the 1,3,6,8-pyrenetetrasulfonate ion are omitted.

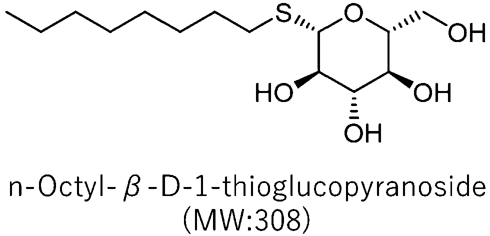

### [Example 4]

Crystals were synthesized in the same manner as in Example 2. From the synthesized crystals, about 50 crystals were taken out together with 10 µL of the mother liquor. To the mother liquor in which the crystals were immersed, 0.1 mg of *N*-(*tert-*butoxycarbonyl)-L-tryptophan was added, and the resulting mixture was left to stand at 20 °C for 12 hours. Subsequently, the immersed crystals were taken out and subjected to crystal structure analysis.

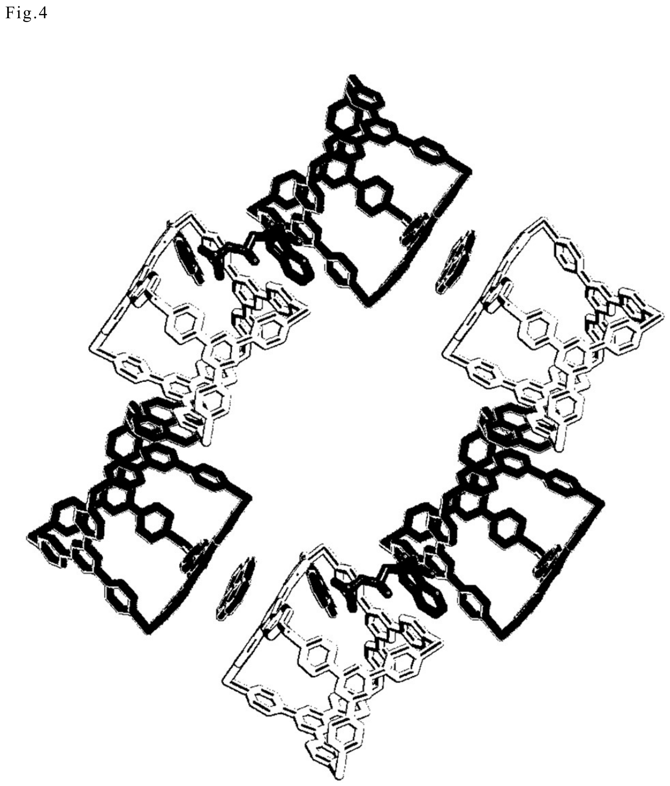

FIG. 4 illustrates the molecular structure obtained by the crystal structure analysis. In FIG. 4, the sulfonate ion groups of the 1,3,6,8-pyrenetetrasulfonate ion are omitted.

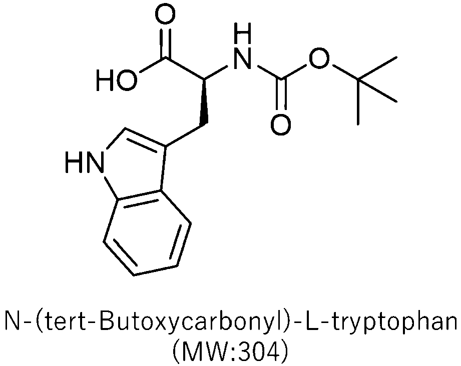

### [Example 5]

To an aqueous solution of nitrate of Host Molecule 2 (concentration, 10 mM; 1 mL), a powder of pyrene in an amount of 5 equivalents with respect to Host Molecule 2 was added to obtain a suspension. The resulting suspension was stirred at 100°C for 10 minutes, and then filtered using a syringe filter. To the filtrate, an aqueous solution of 1,5-naphthalenedisulfonic acid disodium salt (concentration, 20 mM; 1.5 mL; 3 equivalents with respect to Host Molecule 2) was added, and the resulting mixed solution was left to stand at 25°C for 24 hours to to allow precipitation of crystals. The precipitated crystals (length of the long side, about 200 µm) was subjected to crystal structure analysis.

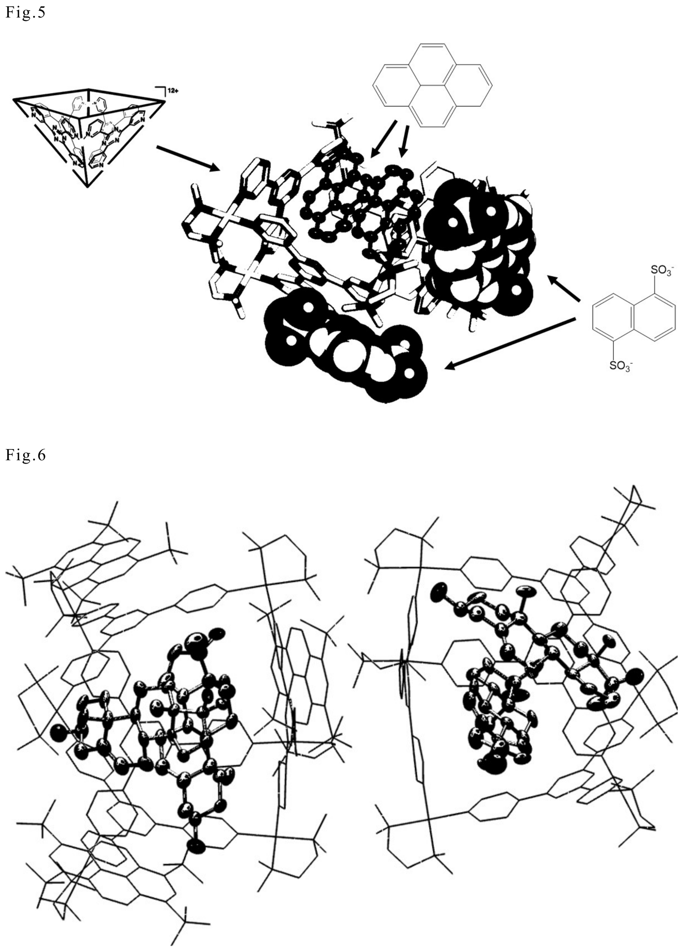

FIG. 5 illustrates the molecular structure obtained by the crystal structure analysis. As illustrated in FIG. 5, two pyrene molecules are accommodated in the internal space of Host Molecule 2. Further, the framework moiety of 1,5-naphthalenedisulfonate ion is oriented almost parallel to a wall portion of Host Molecule 2, and the 1,5-naphthalenedisulfonate ion has affinity interaction with the wall portion of Host Molecule 2.

### [Example 6]

Crystals were synthesized in the same manner as in Example 2. From the synthesized crystals, one crystal was taken out together with 20 µL of the mother liquor. To the mother liquor in which the crystal was immersed, 10 µg of methylandrostenediol was added, and the resulting mixture was left to stand at 20 °C for 12 hours. Subsequently, the immersed crystal was taken out, and subjected to crystal structure analysis.

FIG. 6 illustrates the molecular structure obtained by the crystal structure analysis.

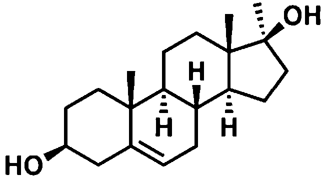

### [Example 7]

To an aqueous solution of nitrate of Host Molecule 1 (concentration, 20 mM; 200 µL), 1 mg of a powder of methylandrostenediol was added to obtain a suspension. The resulting suspension was stirred at 60°C for 15 minutes, and then filtered using a syringe filter. The resulting filtrate (100 µL) was transferred to a round-bottom glass tube having an inner diameter of 6 mm and a length of 40 mm, and an aqueous solution of 1,3,6,8-pyrenetetrasulfonic acid tetrasodium salt (concentration, 20 mM; 20 µL; 0.2 equivalents with respect to Host Molecule 1) and an aqueous agarose solution (0.5% w/w, 100 µL) were added thereto. The resulting mixture was mixed at 60°C and then left to stand at 20°C for 1 hour. Onto this mixture, a mixture of an aqueous agarose solution (0.5% w/w, 100 µL) and an aqueous sodium tetrafluoroborate solution (200 mM, 20 µL) was added, and the mixtures were left to stand for 12 hours to allow precipitation of crystals. Several ten crystals were obtained by the precipitation. Several of these were aspirated together with the gel using a glass pipette, followed by placing the aspirated crystals on a glass plate and mixing the crystals with a protective oil (Paratone). Under visual inspection with a stereomicroscope, one crack-free crystal was selected, mounted on a loop for a single-crystal diffractometer, and set on the diffractometer. An X-ray diffraction image was then obtained. Crystal structure analysis was performed based on the obtained X-ray diffraction image.

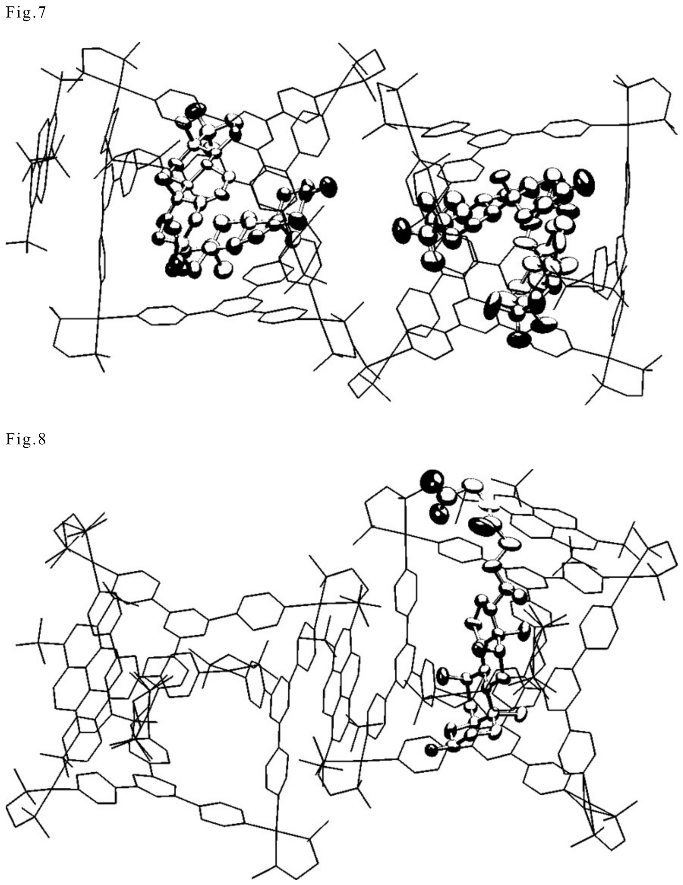

FIG. 7 illustrates the molecular structure obtained by the crystal structure analysis.

### [Example 8]

To an aqueous solution of nitrate of Host Molecule 1 (concentration, 20 mM; 500 µL), 4.9 mg (1 equivalent with respect to Host Molecule 1) of a powder of sodium glycocholate was added to obtain a suspension. The resulting suspension was stirred at 60°C for 15 minutes, and then filtered using a syringe filter. The filtrate (25 µL) was transferred to a round-bottom glass tube having an inner diameter of 6 mm and a length of 40 mm, and an aqueous solution of 1,3,6,8-pyrenetetrasulfonic acid tetrasodium salt (concentration, 20 mM; 5 µL; 0.2 equivalents with respect to Host Molecule 1) and an aqueous agarose solution (0.5% w/w, 50 µL) were added thereto. The resulting mixture was mixed at 60°C and then left to stand at 20°C for 1 hour. A mixture of an aqueous agarose solution (0.5% w/w, 100 µL) and an aqueous sodium tetrafluoroborate solution (400 mM, 40 µL) was heated to 60°C and then placed onto the above mixture, followed by leaving the mixtures to stand for 12 hours to allow precipitation of crystals. Several ten crystals were obtained by the precipitation. Several of these were aspirated together with the gel using a glass pipette, followed by placing the aspirated crystals on a glass plate and mixing the crystals with a protective oil (Paratone). Under visual inspection with a stereomicroscope, one crack-free crystal was selected, mounted on a loop for a single-crystal diffractometer, and set on the diffractometer. An X-ray diffraction image was then obtained. Crystal structure analysis was performed based on the obtained X-ray diffraction image.

FIG. 8 illustrates the molecular structure obtained by the crystal structure analysis.

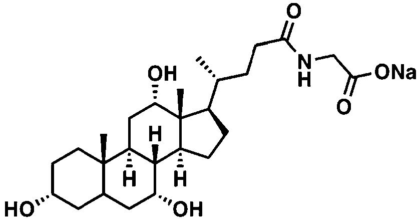

### [Example 9]

To an aqueous solution of nitrate of Host Molecule 1 (concentration, 20 mM; 500 µL), 5.2 mg (1 equivalent with respect to Host Molecule 1) of a powder of beclometasone propionate was added to obtain a suspension. The resulting suspension was stirred at 60°C for 15 minutes, and then filtered using a syringe filter. The filtrate (25 µL) was transferred to a round-bottom glass tube having an inner diameter of 6 mm and a length of 40 mm, and an aqueous solution of 1,3,6,8-pyrenetetrasulfonic acid tetrasodium salt (concentration, 20 mM; 5 µL; 0.2 equivalents with respect to Host Molecule 1) and an aqueous agarose solution (0.5% w/w, 50 µL) were added thereto. The resulting mixture was mixed at 60°C and then left to stand at 20°C for 1 hour. A mixture of an aqueous agarose solution (0.5% w/w, 100 µL) and an aqueous sodium tetrafluoroborate solution (400 mM, 40 µL) was heated to 60°C and then placed onto the above mixture, followed by leaving the mixtures to stand for 12 hours to allow precipitation of crystals. Several ten crystals were obtained by the precipitation. Several of these were aspirated together with the gel using a glass pipette, followed by placing the aspirated crystals on a glass plate and mixing the crystals with a protective oil (Paratone). Under visual inspection with a stereomicroscope, one crack-free crystal was selected, mounted on a loop for a single-crystal diffractometer, and set on the diffractometer. An X-ray diffraction image was then obtained. Crystal structure analysis was performed based on the obtained X-ray diffraction image.

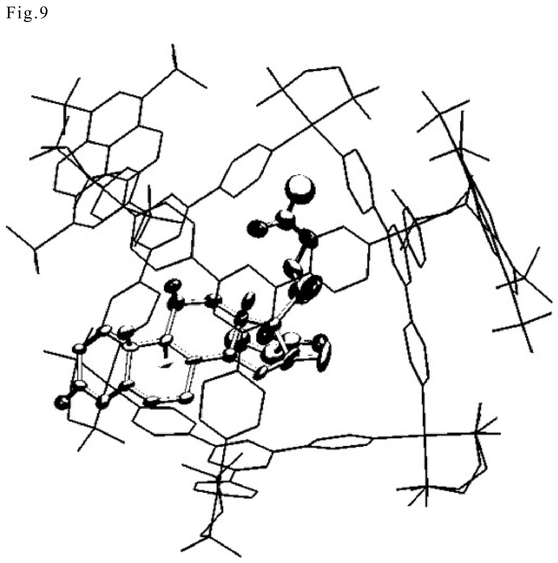

FIG. 9 illustrates the molecular structure obtained by the crystal structure analysis.

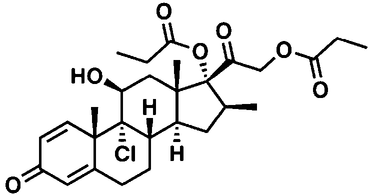

### [Example 10]

To an aqueous solution of nitrate of Host Molecule 1 (concentration, 20 mM; 500 µL), 12.3 mg (1 equivalent with respect to Host Molecule 1) of a powder of digitonin was added to obtain a suspension. The resulting suspension was stirred at 60°C for 15 minutes, and then filtered using a syringe filter. The resulting filtrate (100 µL) was transferred to a round-bottom glass tube having an inner diameter of 6 mm and a length of 40 mm, and an aqueous solution of 1,3,6,8-pyrenetetrasulfonic acid tetrasodium salt (concentration, 20 mM; 20 µL; 0.2 equivalents with respect to Host Molecule 1) and an aqueous agarose solution (0.5% w/w, 100 µL) were added thereto. The resulting mixture was mixed at 60°C and then left to stand at 20°C for 1 hour. A mixture of an aqueous agarose solution (0.25% w/w, 200 µL) and an aqueous sodium tetrafluoroborate solution (400 mM, 40 µL) was heated to 60°C and then placed onto the above mixture, followed by leaving the mixtures to stand for 12 hours to allow precipitation of crystals. Several ten crystals were obtained by the precipitation. Several of these were aspirated together with the gel using a glass pipette, followed by placing the aspirated crystals on a glass plate and mixing the crystals with a protective oil (Paratone). Under visual inspection with a stereomicroscope, one crack-free crystal was selected, mounted on a loop for a single-crystal diffractometer, and set on the diffractometer. An X-ray diffraction image was then obtained. Crystal structure analysis was performed based on the obtained X-ray diffraction image.

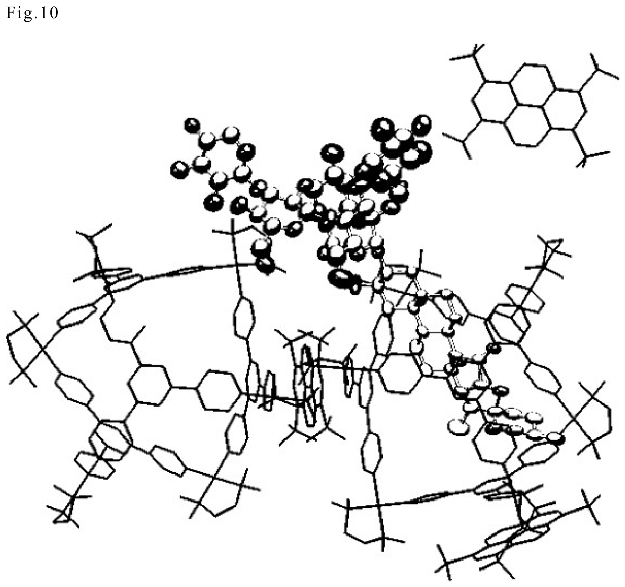

FIG. 10 illustrates the molecular structure obtained by the crystal structure analysis.

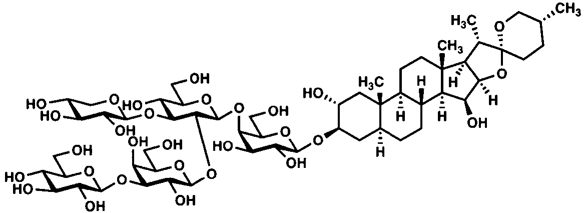

### [Example 11]

To an aqueous solution of nitrate of Host Molecule 1 (concentration, 20 mM; 500 µL), 6.9 mg (1 equivalent with respect to Host Molecule 1) of a powder of monensin sodium salt was added to obtain a suspension. The resulting suspension was stirred at 60°C for 15 minutes, and then filtered using a syringe filter. The filtrate (10 µL) was transferred to a glass capillary tube having an inner diameter of 1 mm, and an aqueous solution of 1,3,6,8-pyrenetetrasulfonic acid tetrasodium salt (concentration, 20 mM; 2 µL; 0.2 equivalents with respect to Host Molecule 1) and an aqueous agarose solution (0.5% w/w, 10 µL) were added thereto. The resulting mixture was mixed at 60°C and then left to stand at 20°C for 1 hour. A mixture of an aqueous agarose solution (0.25% w/w, 20 µL) and an aqueous sodium hexafluoroantimonate solution (100 mM, 2 µL) was heated to 60°C and then added to the above mixture, followed by leaving the mixtures to stand for 12 hours to allow precipitation of crystals. Several ten crystals were obtained by the precipitation. Several of these were aspirated together with the gel using a glass pipette, followed by placing the aspirated crystals on a glass plate and mixing the crystals with a protective oil (Paratone). Under visual inspection with a stereomicroscope, one crack-free crystal was selected, mounted on a loop for a single-crystal diffractometer, and set on the diffractometer. An X-ray diffraction image was then obtained. Crystal structure analysis was performed based on the obtained X-ray diffraction image.

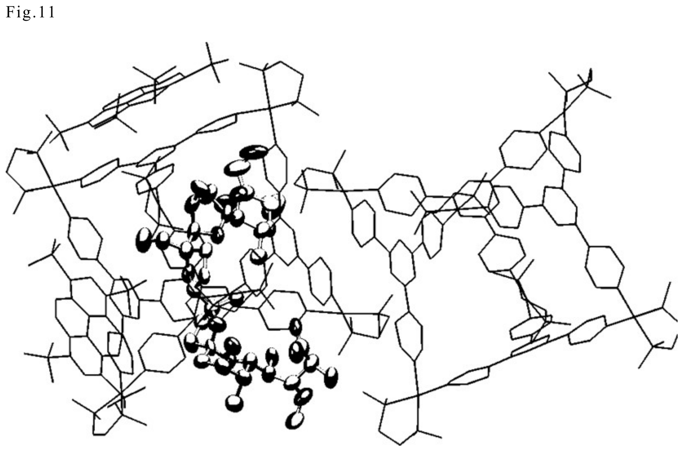

FIG. 11 illustrates the molecular structure obtained by the crystal structure analysis.

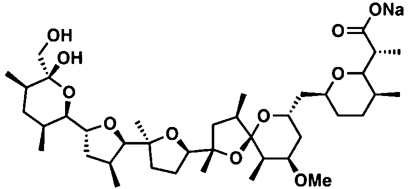

## Claims

1. A sample for crystal structure analysis comprising:
a plurality of host molecules;
a plurality of crystallization-promoting molecules; and
a plurality of target molecules, wherein
each host molecule includes one or more openings, one or more wall portions, and an internal space surrounded by the one or more wall portions,
each crystallization-promoting molecule has a structure capable of affinity interaction with a wall portion of the host molecule,
the plurality of host molecules contained in the sample for crystal structure analysis is three-dimensionally regularly assembled,
each of the plurality of crystallization-promoting molecules contained in the sample for crystal structure analysis is interposed between wall portions of two host molecules,
some or all of the plurality of host molecules contained in the sample for crystal structure analysis accommodate, in the respective internal spaces thereof, a whole target molecule or part of a target molecule, and
the plurality of target molecules contained in the sample for crystal structure analysis is three-dimensionally regularly arranged.

2. The sample for crystal structure analysis according to claim 1, wherein each host molecule is a polynuclear metal complex.

3. The sample for crystal structure analysis according to claim 2, wherein the polynuclear metal complex contains an ion of an element selected from a group consisting of Ti, Fe, Co, Ni, Cu, Zn, Ru, Rh, Pd, Cd, Os, Ir, and Pt, and a multidentate ligand having a π-conjugated system.

4. The sample for crystal structure analysis according to claim 3,
wherein the multidentate ligand having a π-conjugated system is a ligand represented by Formula (1) below: 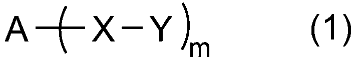
(wherein A is an m-valent group having aromaticity; X is a divalent organic group, or a single bond directly linking A to Y; Y is a coordinating atom, or a monovalent group containing a coordinating atom; m represents an integer of 2 to 6; a plurality of Xs may be different from each other; and a plurality of Ys may be different from each other).

5. The sample for crystal structure analysis according to claim 1,
wherein the affinity interaction between the crystallization-promoting molecule and the wall portion of the host molecule is hydrophobic interaction, π-π interaction, or CH-π interaction.

6. The sample for crystal structure analysis according to claim 1,
wherein the crystallization-promoting molecule is a molecule having a π-conjugated system.

7. The sample for crystal structure analysis according to claim 1,
wherein the crystallization-promoting molecule is a molecule containing a fused aromatic ring.

8. The sample for crystal structure analysis according to claim 1,
wherein the crystallization-promoting molecule is a molecule containing an anionic group.

9. The sample for crystal structure analysis according to claim 1, wherein
an overall charge of each host molecule is a positive charge, and an overall charge of each crystallization-promoting molecule is a negative charge; or
an overall charge of each host molecule is a negative charge, and an overall charge of each crystallization-promoting molecule is a positive charge.

10. A sample precursor for crystal structure analysis, comprising a plurality of host molecules and a plurality of crystallization-promoting molecules, wherein
each host molecule includes one or more openings, one or more wall portions, and an internal space surrounded by the one or more wall portions,
each crystallization-promoting molecule has a structure capable of affinity interaction with a wall portion of the host molecule,
the plurality of host molecules contained in the sample precursor for crystal structure analysis is three-dimensionally regularly assembled, and
each of the plurality of crystallization-promoting molecules contained in the sample precursor for crystal structure analysis is interposed between wall portions of two host molecules.

11. A method of producing the sample for crystal structure analysis according to claim 1, comprising a step of allowing the sample precursor for crystal structure analysis according to claim 10 to coexist with target molecules in a same system in presence of a solvent or absence of a solvent to accommodate the respective target molecules in the internal spaces of some or all of the host molecules contained in the sample precursor for crystal structure analysis.

12. A method of producing the sample for crystal structure analysis according to claim 1, comprising steps of:
allowing host molecules each including one or more openings, one or more wall portions, and an internal space surrounded by the one or more wall portions to coexist with target molecules in a same system in presence of a solvent or absence of a solvent to accommodate the target molecules in the internal spaces of the host molecules (Step a-I);
adding crystallization-promoting molecules to the solution obtained by performing Step a-I in the presence of the solvent, to prepare a solution containing: the host molecules accommodating the target molecules; and the crystallization-promoting molecules (Step a-II); or dissolving the host molecules accommodating the target molecules produced by performing Step a-I in the absence of the solvent, and crystallization-promoting molecules, in a solvent to prepare a solution containing: the host molecules accommodating the target molecules; and the crystallization-promoting molecules (Step a-III); and
precipitating a single crystal containing: the host molecules accommodating the target molecules; and the crystallization-promoting molecules; from the solution obtained in Step a-II or Step a-III (Step a-IV).

13. A method of producing the sample for crystal structure analysis according to claim 1, comprising steps of:
dissolving host molecules each including one or more openings, one or more wall portions, and an internal space surrounded by the one or more wall portions; target molecules; and crystallization-promoting molecules; in a solvent to prepare a mixed solution (Step b-I); and
precipitating a single crystal containing: host molecules accommodating the target molecules; and the crystallization-promoting molecules; from the mixed solution obtained in Step b-I (Step b-II).

14. A kit for preparing a sample for crystal structure analysis, comprising a combination of: a host molecule including one or more openings, one or more wall portions, and an internal space surrounded by the one or more wall portions; and a crystallization-promoting molecule having a structure capable of affinity interaction with a wall portion of the host molecule.
